# EUROPEAN PATENT SPECIFICATION

(11) **EP 4 541 296 B1**
(45) Date of publication and mention of the grant of the patent: **25.03.2026**
(21) Application number: 23203902.4
(22) Date of filing: 16.10.2023
(51) Int. Cl.: A61B 17/15, A61B 17/88

(54) **PATELLA HOLDING DEVICE**
PATELLAHALTEVORRICHTUNG
DISPOSITIF DE MAINTIEN DE ROTULE

(43) Date of publication of application: 23.04.2025
(73) Proprietor: Aesculap AG, 78532 Tuttlingen (DE)
(72) Inventor: Mattes, Ursula, 78603 Renquishausen (DE); Snauwaert, Eliott, 52000 Chaumont (FR)
(74) Representative: DREISS Patentanwälte PartG mbB

(56) References cited:
- CN-A- 112 370 145
- US-A1- 2012 101 505

## Description

The invention relates to a patella holding device for holding a patella in a holding plane as defined in claim a prior art patella holding device is for example disclosed in US 2006/0161165 A1.

As a part of a total knee replacement or total knee arthroplasty ("TKA") surgery, the posterior portion of a patella may be resected for implanting with a patellar knee component. The step of resecting the patella requires the use of a patella holding device for retaining or holding the patella during cutting. US 2006/0161165 A1 proposes to provide a pincer-like structure with two mutually movable handle parts, one handle part being connected to a first clamping section engaging the skin / subcutaneous tissues overlying the anterior of a patella and the other handle part being connected to a second clamping section engaging the posterior of a patella, thus providing a clamping action along an antero-posterior axis of the patella.

CN 112 370 145 A discloses a patella holding forceps comprising first and second inlays, each inlay comprising a clamping chain for holding the patella.

One object of the present invention is to provide a patella holding device which allows for a reliable holding of a patella and for a comfortable handling by a surgeon.

This object is solved by the patella holding device with the features of claim 1. Preferred embodiments are defined in the dependent claims.

According to the invention, the patella holding device comprises two gripping elements which are connected to each other via a chain. The chain comprises chain links, wherein adjacent chain links are movable relative to each other about a rotation axis which is perpendicular to the holding plane.

The chain elements provide a flexible structure for surrounding a holding zone of a patella. This flexibility ensures a reliable fit of the patella holding device and the patella which is independent of the size and shape of the patella. At the same time, the chain elements provide for a connection of the two gripping elements allowing for a comfortable handling by a surgeon.

According to a preferred embodiment, the holding plane is parallel or coincides with a plane defined by the medio-lateral axis and the proximal-distal axis of the patella.

In particular, the holding plane is parallel to or coincides with a resection plane of the patella. The chain elements surrounding the holding zone of the patella are arranged within the holding plane and can be positioned such that the two gripping elements are arranged adjacent to each other. The gripping elements can be located at any angular position within the holding plane. For example, the gripping elements can be arranged on a proximal, a lateral, a distal or a medial side of a patella and in any angular intermediate position, in accordance with the preference of a surgeon. In addition, a surgeon can use the patella holding device in a first orientation with respect to the holding plane and in a reverse orientation with respect to the holding plane.

In an embodiment, the chain comprises at least 8 pivot axes, preferably at least 12 rotation axes, in particular at least 16 pivot axes. A higher number of pivot axes will allow for a snug fit of the chain along the circumference of a holding zone of the patella.

In one embodiment, the chain comprises at most 20 pivot axes, limiting the number of parts to be assembled during production and the number of parts to be cleaned after use.

In one embodiment, the chain comprises chain links which are configured as outer links and chain links which are configured as inner links, wherein the outer links and the inner links are arranged in an alternate manner, thus providing a stable and space saving structure.

According to a preferred embodiment, at least some of the chain links have first outer surfaces which together define a support plane for supporting a resection tool. Such a resection tool can be a saw blade which is supported by the outer surfaces. Preferably, the resection tool has a width which is great enough for the resection tool to be supported by at least two first outer surfaces at the same time, for example by at least two first outer surfaces of two adjacent outer chain links.

According to a further preferred embodiment, at least some of the chain links have second outer surfaces which together define a second support plane for supporting a resection tool, the second outer surfaces being parallel to the first outer surfaces. In this way, a support plane for a resection tool can be provided independent of the first surfaces or the second surfaces being arranged at the level of a resection plane (in other words: a support plane is provided in a first orientation and in a reverse orientation of the patella holding device).

To improve a holding effect of the patella holding device, at least some of the chain links comprise one protrusion or at least one protrusion which is arranged within the holding plane and configured to engage with the patella. For example, the protrusions are arranged at inner links of the chain. The at least one protrusion provides an anchoring element or fixing element and may for example have a sharp corner, such as a tooth or a hook.

According to a preferred embodiment, a first gripping element is configured to be manipulated by a thumb of a hand of a surgeon and in that a second gripping element is configured to be manipulated by an index finger, in particular of the same hand of the surgeon. A surgeon may use his right or left hand in accordance with his preference. In a preferred embodiment, the two gripping elements have an identical shape and/or size.

To provide a comfortable handling, the gripping elements may be ring-shaped and/or trough-shaped. In a preferred embodiment, the two gripping elements have an identical shape and/or size.

Further features and advantages are the subject of the following description and of the diagrammatic illustration of embodiments.

In the drawings:
- Figure 1: shows a perspective view of one embodiment of a patella holding device;
- Figure 2: shows a perspective view of an initial engagement of the holding device with a patella;
- Figure 3: shows a perspective view of a complete engagement of the holding device with the patella; and
- Figure 4: shows a perspective view of the configuration according to Fig. 3 and of a resection tool.

According to the drawings, a patella holding device 10 comprises two trough-shaped gripping elements 12 and 14. Each of the gripping elements may be manipulated by placing the top of a thumb or the top of an index finger of a surgeon into the trough. In particular, a surgeon may use the thumb and an index finger of the same hand.

The gripping elements 12 and 14 are connected to each other by means of a chain 16. The chain 16 comprises outer chain links 18 and inner chain links 20 which are arranged in an alternate manner. For example, the chain comprises 10 outer chain links 18 and 9 inner chain links 20. For example, each of the gripping elements 12 and 14 is connected to an outer chain link 18, preferably in a non-movable manner.

Adjacent links 18 and 20 are connected to and movable with respect to each other by means of chain pins 22, each chain pin 22 providing a rotational axis 24 around which adjacent chain links 18 and 20 can rotate or swivel within a holding plane 26 of the patella holding device 10.

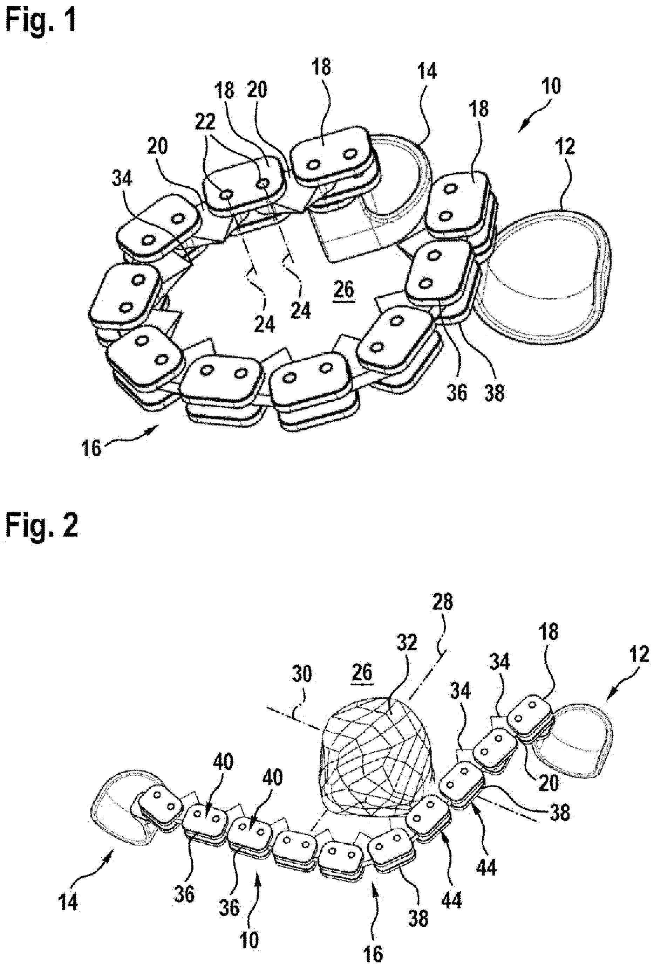

Fig. 2 illustrates an initial holding action of the patella holding device 10 within the holding plane 26 which is defined by a medio-lateral axis 28 and a proximal-distal axis 30 of a patella 32. The proximal-distal axis 30 is oriented in parallel with the main orientation of a patellar tendon (not shown in the drawings).

The inner chain links 20 have a width as seen in a direction parallel to the rotation axes 24 which is smaller than the width of the outer chain links 18.

Preferably, the inner chain links 20 are each provided with one tooth-shaped protrusion 34 which is configured to engage with an engaging zone along the circumference of the patella 32.

Preferably, each outer chain links 18 comprises two plates or tabs 36, 38 which are spaced from another and overlap and adjacent inner chain link 18, see Fig. 2.

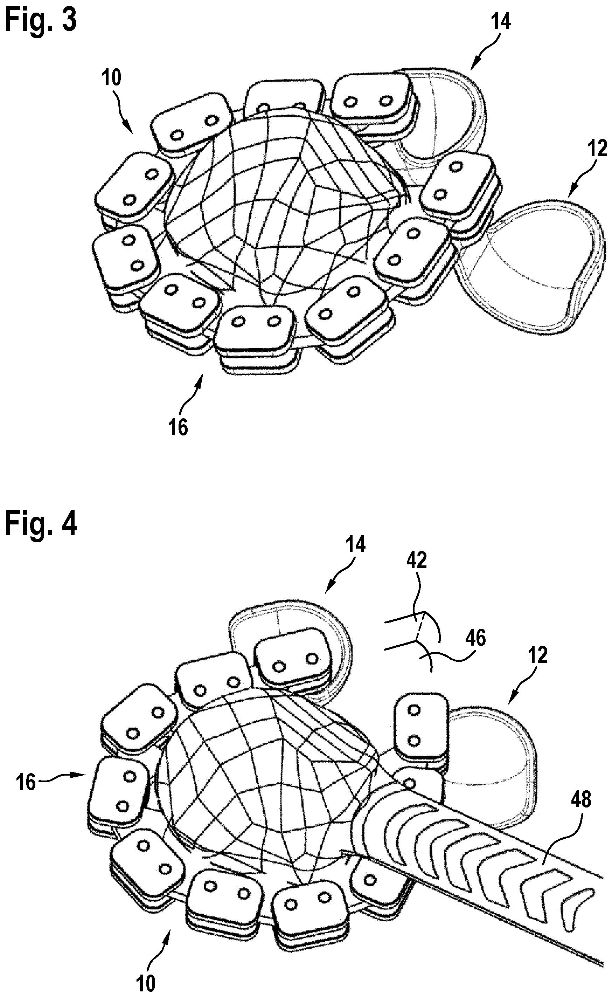

The tabs 36 of the outer chain links 18 each have a first outer surface 40, the plurality of outer surfaces 38 defining a first support plane 42 (schematically illustrated in Fig. 4).

The tabs 38 of the outer chain links 18 each have a second outer surface 44, the plurality of outer surfaces 44 defining a second support plane 46 (schematically illustrated in Fig. 4).

The support planes 42, 46 allow the support and guidance of a resection tool 48, for example a saw blade, see Fig. 4, wherein the resection tool 48 is supported by the first support plane 42.

In order to hold the patella 32 a surgeon may place the chain 16 and its chain links 18, 20 within the holding plane 26 and around the circumference of the patella 32, see Fig. 3. The two gripping elements 18, 20 may be positioned at any angular position within the clamping plane 34 (360 degrees of freedom).

The patella holding device 10 may also be used in a reverse configuration in which the gripping elements 18, 20 face into an opposite direction and in which the resection tool 48 would be supported by the second support plane 46.

## Claims

1. Patella holding device (10) for holding a patella (32) in a holding plane (26), wherein the patella holding device (10) comprises two gripping elements (12, 14) which are connected to each other via a chain (16) which comprises chain links (18, 20), wherein adjacent chain links (18, 20) are movable relative to each other about a rotation axis (24) which is perpendicular to the holding plane (26).

2. Patella holding device (10) according to claim 1, **characterized in that** the holding plane (26) is parallel or coincides with a plane defined by the medio-lateral axis (30) and the proximal-distal axis (28) of the patella (32).

3. Patella holding device (10) according to one of the preceding claims, **characterized in that** the chain (16) comprises at least 8 pivot axes (24), preferably at least 12 rotation axes (24), in particular at least 16 pivot axes (24).

4. Patella holding device (10) according to one of the preceding claims, **characterized in that** the chain (16) comprises at most 20 pivot axes (24).

5. Patella holding device (10) according to one of the preceding claims, **characterized in that** the chain (16) comprises chain links (18) which are configured as outer links and chain links (20) which are configured as inner links, wherein the outer links and the inner links are arranged in an alternate manner.

6. Patella holding device (10) according to one of the preceding claims, **characterized in that** at least some of the chain links (18) have first outer surfaces (40) which together define a support plane (42) for supporting a resection tool (48).

7. Patella holding device (10) according to claim 6, **characterized in that** at least some of the chain links (18) have second outer surfaces (44) which together define a second support plane (46) for supporting a resection tool, the second outer surfaces (46) being parallel to the first outer surfaces (40).

8. Patella holding device (10) according to one of the preceding claims, **characterized in that** at least some of the chain links (20) comprise one protrusion (34) or at least one protrusion (34) which is arranged within the holding plane (26) and configured to engage with the patella (32).

9. Patella holding device (10) according to one of the preceding claims, **characterized in that** a first gripping element (12) is configured to be manipulated by a thumb of a hand of a surgeon and **in that** a second gripping element (14) is configured to be manipulated by an index finger, in particular of the same hand of the surgeon.

10. Patella holding device (10) according to one of the preceding claims, **characterized in that** the gripping elements (12, 14) are ring-shaped and/or trough-shaped.

## Patentansprüche

1. Patella-Haltevorrichtung (10) zum Halten einer Patella (32) in einer Halteebene (26), wobei die Patella-Haltevorrichtung (10) zwei Greifelemente (12, 14) umfasst, die über eine Kette (16) miteinander verbunden sind, die Kettenglieder (18, 20) umfasst, wobei angrenzende Kettenglieder (18, 20) relativ zueinander um eine Drehachse (24) herum bewegbar sind, die senkrecht zu der Halteebene (26) ist.

2. Patella-Haltevorrichtung (10) nach Anspruch 1,
**dadurch gekennzeichnet, dass** die Halteebene (26) parallel ist oder mit einer Ebene zusammenfällt, die durch die medio-laterale Achse (30) und die proximaldistale Achse (28) der Patella (32) definiert ist.

3. Patella-Haltevorrichtung (10) nach einem der vorstehenden Ansprüche, **dadurch gekennzeichnet, dass** die Kette (16) mindestens 8 Schwenkachsen (24), vorzugsweise mindestens 12 Rotationsachsen (24), insbesondere mindestens 16 Schwenkachsen (24) umfasst.

4. Patella-Haltevorrichtung (10) nach einem der vorstehenden Ansprüche, **dadurch gekennzeichnet, dass** die Kette (16) höchstens 20 Schwenkachsen (24) umfasst.

5. Patella-Haltevorrichtung (10) nach einem der vorstehenden Ansprüche, **dadurch gekennzeichnet, dass** die Kette (16) Kettenglieder (18), die als Außenglieder konfiguriert sind, und Kettenglieder (20) umfasst, die als Innenglieder konfiguriert sind, wobei die Außenglieder und die Innenglieder in einer abwechselnden Weise angeordnet sind.

6. Patella-Haltevorrichtung (10) nach einem der vorstehenden Ansprüche, **dadurch gekennzeichnet, dass** mindestens einige der Kettenglieder (18) erste Außenoberflächen (40) aufweisen, die zusammen eine Stützebene (42) zum Stützen eines Resektionswerkzeugs (48) definieren.

7. Patella-Haltevorrichtung (10) nach Anspruch 6,
**dadurch gekennzeichnet, dass** mindestens einige der Kettenglieder (18) zweite Außenoberflächen (44) aufweisen, die zusammen eine zweite Stützebene (46) zum Stützen eines Resektionswerkzeugs definieren, wobei die zweiten Außenoberflächen (46) parallel zu den ersten Außenoberflächen (40) sind.

8. Patella-Haltevorrichtung (10) nach einem der vorstehenden Ansprüche, **dadurch gekennzeichnet, dass** mindestens einige der Kettenglieder (20) einen Vorsprung (34) oder mindestens einen Vorsprung (34), der innerhalb der Halteebene (26) angeordnet und konfiguriert ist, um mit der Patella (32) in Eingriff zu stehen, umfassen.

9. Patella-Haltevorrichtung (10) nach einem der vorstehenden Ansprüche, **dadurch gekennzeichnet, dass** ein erstes Greifelement (12) konfiguriert ist, um durch einen Daumen einer Hand eines Chirurgen manipuliert zu werden, und dass ein zweites Greifelement (14) konfiguriert ist, um durch einen Zeigefinger, insbesondere derselben Hand des Chirurgen, manipuliert zu werden.

10. Patella-Haltevorrichtung (10) nach einem der vorstehenden Ansprüche, **dadurch gekennzeichnet, dass** die Greifelemente (12, 14) ringförmig und/oder muldenförmig sind.

## Revendications

1. Dispositif de maintien de rotule (10) pour maintenir une rotule (32) dans un plan de maintien (26), dans lequel le dispositif de maintien de rotule (10) comprend deux éléments de préhension (12, 14) qui sont reliés l'un à l'autre par l'intermédiaire d'une chaîne (16) qui comprend des maillons de chaîne (18, 20), dans lequel des maillons de chaîne adjacents (18, 20) sont mobiles l'un par rapport à l'autre autour d'un axe de rotation (24) qui est perpendiculaire au plan de maintien (26).

2. Dispositif de maintien de rotule (10) selon la revendication 1,
**caractérisé en ce que** le plan de maintien (26) est parallèle ou coïncide avec un plan défini par l'axe médio-latéral (30) et l'axe proximal/distal (28) de la rotule (32).

3. Dispositif de maintien de rotule (10) selon l'une des revendications précédentes, **caractérisé en ce que** la chaîne (16) comprend au moins 8 axes de pivotement (24), de préférence au moins 12 axes de rotation (24), en particulier au moins 16 axes de pivotement (24).

4. Dispositif de maintien de rotule (10) selon l'une des revendications précédentes, **caractérisé en ce que** la chaîne (16) comprend au plus 20 axes de pivotement (24).

5. Dispositif de maintien de la rotule (10) selon l'une des revendications précédentes, **caractérisé en ce que** la chaîne (16) comprend des maillons de chaîne (18) conçus comme maillons extérieurs et des maillons de chaîne (20) conçus comme maillons intérieurs, dans lequel les maillons extérieurs et les maillons intérieurs sont agencés de manière alternée.

6. Dispositif de maintien de rotule (10) selon l'une des revendications précédentes, **caractérisé en ce qu'**au moins certains des maillons de la chaîne (18) présentent des premières surfaces extérieures (40) qui définissent ensemble un plan de support (42) pour supporter un outil de résection (48).

7. Dispositif de maintien de rotule (10) selon la revendication 6,
**caractérisé en ce qu'**au moins certains des maillons de chaîne (18) présentent des secondes surfaces extérieures (44) qui définissent ensemble un second plan de support (46) pour supporter un outil de résection, les secondes surfaces extérieures (46) étant parallèles aux premières surfaces extérieures (40).

8. Dispositif de maintien de rotule (10) selon l'une des revendications précédentes, **caractérisé en ce qu'**au moins certains des maillons de chaîne (20) comprennent une saillie (34) ou au moins une saillie (34) qui est agencée dans le plan de maintien (26) et conçue pour être en prise avec la rotule (32).

9. Dispositif de maintien de rotule (10) selon l'une des revendications précédentes, **caractérisé en ce qu'**un premier élément de préhension (12) est conçu pour être manipulé par un pouce d'une main d'un chirurgien et **en ce qu'**un second élément de préhension (14) est conçu pour être manipulé par un index, en particulier de la même main du chirurgien.

10. Dispositif de maintien de rotule (10) selon l'une des revendications précédentes, **caractérisé en ce que** les éléments de préhension (12, 14) sont en forme d'anneau et/ou d'auge.
